# EUROPÄISCHE PATENTSCHRIFT

(11) **EP 1 293 494 B1**
(45) Veröffentlichungstag und Bekanntmachung des Hinweises auf die Patenterteilung: **03.09.2003**
(21) Anmeldenummer: 02019099.7
(22) Anmeldetag: 29.08.2002
(51) Int. Cl.: C07C 45/63

(54) **Verfahren zur Herstellung von alpha-Halogenketonen**
Process for preparing alpha-halo-ketones
Procédé de préparation de cétones aplha-halogénées

(30) Priorität: 06.09.2001 DE 10143742
(43) Veröffentlichungstag der Anmeldung: 19.03.2003
(73) Patentinhaber: Consortium für elektrochemische Industrie GmbH, 81379 München (DE)
(72) Erfinder: Heldmann, Dieter, Dr., 81379 München (DE); Stohrer, Jürgen, Dr., 82049 Pullach (DE)
(74) Vertreter: Potten, Holger

(56) Entgegenhaltungen:
- EP-A- 0 774 453
- US-A- 5 929 284

## Beschreibung

Die Erfindung betrifft ein Verfahren zur Herstellung von α-Halogenketonen.

Die Herstellung von α-Chlorketonen durch Umsetzung einer N-geschützten Aminosäure mit Chlorameisensäurealkylestern zum gemischten Anhydrid, Umsetzung des gemischten Anhydrids mit Diazomethan zum Diazoketon und nachfolgender Reaktion mit HCl zum Chlorketon ist z. B. aus S. W. Kaldor et al., J. Med. Chem. 1997, 40, 3979-3985 bekannt. Das Verfahren verwendet Diazomethan, ein explosives und cancerogenes Gas, das im technischen Maßstab nur unter großen Gefahren verwendet werden kann.
Die Herstellung von α-Chlorketonen durch Umsetzung eines N-geschützten Aminosäureesters mit einem CH₂Cl-Anion bei tiefen Temperaturen ist z. B. aus P. Chen et al, Tetrahedron Lett.
1997, 38(18), 3175-3178 oder US 5,481,011; US 5,523,463; US 5,591,885 bekannt. Das Verfahren muss bei sehr tiefen Temperaturen (T < -80°C) durchgeführt werden, was die allgemeine technische Anwendbarkeit einschränkt und deutliche Kostennachteile mit sich bringt.
Die Herstellung von α-Chlorketonen durch Umsetzung eines N-geschützten Aminosäureesters mit Salzen der Chloressigsäure und nachfolgender Decarboxylierung ist z. B. aus X. Wang et al., Synlett 2000, 902-904 oder US 5,929,284 bekannt. Für Li-Salze ist hier wieder eine technische Tieftemperaturapparatur notwendig. Bei Magnesiumsalzen kann die Reaktion bei Raumtemperatur (RT) und höher durchgeführt werden, die Reaktion liefert dann jedoch nach Chromatographie nur 52% Ausbeute.

Die Herstellung von α-Chlorketonen durch Umsetzung einer aktivierten N-geschützten Aminosäure mit Alkalimetall-Enolaten von Acetaten zu β-Ketoesterderivaten die nachfolgend in einem zweiten Schritt selektiv in 2-Position chloriert werden und in einem abschließenden 3. Schritt decarboxyliert werden ist aus EP 774 453, US 5,767,316 oder US 5,902,887 bekannt. Das Verfahren benötigt 3 Stufen und ist deshalb aufwändiger als die bisher beschriebenen Verfahren.
Die Herstellung von α-Chlorketonen durch Umsetzung einer aktivierten N-geschützten Aminosäure mit Alkalimetall-Enolaten von Monohalogenacetaten zu halogenierten β-Ketoesterderivaten, die dann in Folgeschritten hydrolysiert und decarboxyliert werden, ist in US 5,767,316; US 5,902,887 beschrieben. Das Verfahren muss nach Erfinderangaben (EP 774 453 A1, S. 5, Z. 46-47) bei -60°C oder weniger durchgeführt werden, was wiederum eine technische Tieftemperaturanlage bedingt.

Aufgabe der vorliegenden Erfindung ist es, ein preisgünstiges Verfahren zur Herstellung eines α-Halogenketons aus einem N-geschützten Aminosäurederivat zur Verfügung zu stellen, das gefahrlos in technischem Maßstab durchgeführt werden kann und das bessere Ausbeuten und Reinheiten als die bekannten Verfahren liefert.

Die Aufgabe wird gelöst durch ein Verfahren zur Herstellung eines α-Halogenketons der allgemeinen Formel (1) 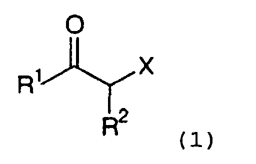 wobei R¹ ein Alkyl-, Aralkyl- oder Arylrest ist, bei dem gegebenenfalls CH₂-Einheiten durch Heteroatome wie NH, NCH₃, S, O substituiert sein können und gegebenenfalls CH-Einheiten durch N substituiert sein können und die Reste R¹ ferner mit funktionellen Gruppen wie z.B. einem Halogenrest, einem Aminorest, einem Alkoxyrest oder einem Thioalkylrest substituiert sein können und
R² ein Wasserstoff-, Alkyl-, Aralkyl- oder Arylrest ist und
X ein Halogenrest ist
in welchem ein Carbonsäurederivat der allgemeinen Formel (2) 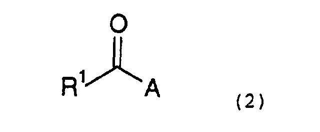 wobei R¹ die bereits genannte Bedeutung hat und
A eine Abgangsgruppe ist
mit einem Mono- oder Dienolat eines Silylesters der allgemeinen Formel (3) 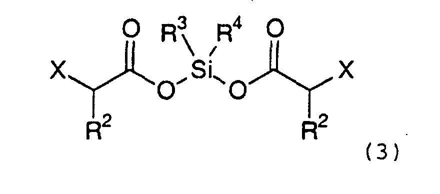 wobei X und R² die bereits genannte Bedeutung haben und
R³, R⁴ gleich oder verschieden sind und Wasserstoff, Alkyl-,
Aryl-, Alkenyl- oder Aralkylrest bedeuten;
zur Reaktion gebracht wird und nach erfolgter Umsetzung das Reaktionsprodukt ohne Isolierung unmittelbar anschließend durch Säurezusatz hydrolysiert und zu (1) decarboxyliert wird.

Unter einer Abgangsgruppe ist vorzugsweise ein solcher Rest zu verstehen, der in einem Carbonsäurederivat eine erhöhte Reaktivität gegenüber Nucleophilen im Vergleich zur freien Carbonsäure bewirkt und durch diese Nucleophile eine Substitution am Carbonylrest des Carbonylsäurederivats fördert.

Vorzugsweise ist R¹ ausgewählt aus der Gruppe linearer oder verzweigter Alkylrest mit 1 bis 25 Kohlenstoffatomen, Arylrest mit 6 bis 30 Kohlenstoffatomen, Aralkylrest mit 7 bis 31 Kohlenstoffatomen, in denen gegebenenfalls CH₂-Einheiten durch Heteroatome wie NH, NCH₃, S, O substituiert sein können, und CH-Einheiten durch N substituiert sein können, wobei die Reste R¹ mit funktionellen Gruppen wie z.B. Halogenrest, Aminorest, Alkoxyrest oder Thioalkylrest substituiert sein können.

Beispiele für R¹ sind Methyl-, Ethyl-, Propyl-, Butyl-, Pentyl, Hexyl, Heptyl, Octyl-, Nonyl, Isopropyl-, Isobutyl-, t-Butyl-, 2,2-Dimethylpropyl-, 3-Methylbutyl-, Phenyl-, Naphthyl-, Benzylrest.

Die Abgangsgruppe A ist vorzugsweise ausgewählt aus der Gruppe Alkoxyrest mit 1 bis 10 Kohlenstoffatomen, gegebenenfalls am Ring substituierter Phenoxy- oder Benzyloxyrest, Halogenrest wie Brom- oder Chlorrest, Imidazolylrest, 1-Oxybenzotriazol, Alkoxycarbonyloxy-Gruppen wie Methoxycarbonyloxy, Ethylcarbonyloxy, Isobutoxycarbonyloxy (sog. gemischte Anhydride).

Bevorzugt handelt es sich bei der Abgangsgruppe A um einen linearen Alkoxyrest mit 1 bis 4 C-Atomen.

Besonders bevorzugt ist als Abgangsgruppe A der Methoxy-Rest.

X bedeutet Cl, Br, F oder I, besonders bevorzugt Cl und Br, insbesondere bevorzugt Cl.

Bei dem Rest R² handelt es sich vorzugsweise unabhängig voneinander um einen Wasserstoffrest, Alkylrest mit 1 bis 10 Kohlenstoffatomen, Arylrest mit 6 bis 10 Kohlenstoffatomen, Aralkylrest von 7 bis 11 Kohlenstoffatomen oder einen Alkenylrest mit 2 bis 10 Kohlenstoffatomen.
Bevorzugter Rest R² ist der Wasserstoffrest.

Bei den Resten R³ und R⁴ handelt es sich vorzugsweise unabhängig voneinander um einen Wasserstoffrest, Alkylrest mit 1 bis 10 Kohlenstoffatomen, Arylrest mit 6 bis 10 Kohlenstoffatomen, oder einen Alkenylrest mit 2 bis 10 Kohlenstoffatomen. Beispiele für Silylreste SiR³R⁴ sind der Dimethylsilyl, Diethylsilyl, Diisopropylsilyl, Di-(t-Butyl)silyl, Dibutylsilyl, t-Butylmethylsilyl, Phenylmethylsilyl, Diphenylsilyl, Divinylsilylrest.
Bevorzugt sind dabei der Dimethylsilyl- und Diphenylsilylrest. Als Silylrest besonders bevorzugt ist der Dimethylsilylrest.

Das erfindungsgemäße Verfahren wird vorzugsweise bei einer Temperatur im Bereich von -10°C bis 110°C, bevorzugt bei 0°C bis 70°C durchgeführt.

Das erfindungsgemäße Verfahren umfasst nur einen Verfahrensschritt, benötigt keine Tieftemperaturanlage, keine gefährlichen Chemikalien, verwendet kostengünstige Silylreste und liefert bessere Ausbeuten als bekannte Verfahren.

Das erfindungsgemäße Verfahren wird vorzugsweise in Gegenwart eines Lösungsmittels durchgeführt. Als Lösungsmittel finden neben aromatischen Kohlenwasserstoffen wie Benzol, Toluol oder Xylol sowie Hexan, Heptan bevorzugt Ether wie t-Butyl-methylether, Tetrahydrofuran, Dioxan, Diethylether, Diisopropylether, Dibutylether und 1,2-Dimethoxyethan Verwendung.

Völlig überraschend zeigte sich, dass ein Bis-(α-Halogencarbonsäure)silylester bei Raumtemperatur oder sogar erhöhter Temperatur mit speziellen Basen stabile Enolate bilden kann, die sich in der gewünschten Weise an aktivierte (Amino)-Säurederivate addieren und bei saurer Aufarbeitung durch unmittelbare Hydrolyse und in-situ-Decarboxylierung die gewünschten α-Halogenketone ergeben. Gemäß Stand der Techik war damit zu rechnen daß Enolate von Halogenessigsäuresilylestern nur bei sehr tiefen Temperaturen (T < -60°C) und dann auch nur mit geringen Umsätzen bis etwa 50% zur Herstellung von Chlorketonen geeignet sind.

Die vorliegende Erfindung betrifft insbesondere ein Verfahren zur Herstellung eines α-Halogenketons der allgemeinen Formel (5) 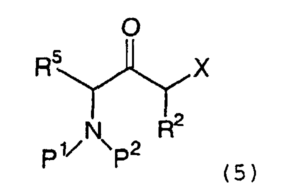 wobei R², wie oben definiert ist, und R⁵ Wasserstoff-, Alkyl-, Aralkyl- oder Arylrest bedeutet, bei dem eine oder mehrere CH₂-Gruppen durch O, S, NH, NCH₃ substituiert sein können, und
X ein Halogenrest ist und
P¹, und P² unabhängig voneinander Wasserstoffrest oder eine Amino-Schutzgruppe sind, bzw. zusammengenommen eine cyclische Aminoschutzgruppe sind
bei dem ein Aminosäurederivat der allgemeinen Formel (6) 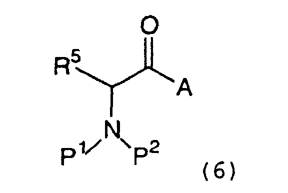 wobei R⁵, P¹, P² die bereits genannte Bedeutung haben und A eine Abgangsgruppe ist, die wie oben definiert ist,
mit einem Metallenolat eines Silylesters der allgemeinen Formel (3) zur Reaktion gebracht wird und das Reaktionsprodukt anschließend unmittelbar durch einen Säurezusatz hydrolysiert und zu (5) decarboxyliert wird.

R⁵ ist vorzugsweise ausgewählt aus der Gruppe Wasserstoffrest, linearer oder verzweigter Alkylrest mit 1 bis 20 C-Atomen, bei dem eine oder mehrere CH₂-Gruppen durch O, S, NH, NCH₃ substituiert sein können, Arylrest mit 6 bis 25 Kohlenstoffatomen, Aralkylrest von 7 bis 26 Kohlenstoffatomen, optional substituierter Vinyl- und Alkinylrest und der Seitenkette einer Aminosäure oder eines Aminosäurederivates, das durch chemische Modifikation der Seitenkette R¹ einer (un-)natürlichen Aminosäure erhalten werden kann.

Beispiele für R⁵ sind Wasserstoff, Methyl-, Ethyl-, Isopropyl-, t-Butyl-, Hydroxymethyl-, Chlormethyl-, 1-Hydroxyethyl-, Mercaptomethyl-, Methylthiomethyl-, Methylthioethyl-, 2-Methylpropyl-, 1-Methylpropyl-, 1-Hydroxyethyl, Phenylthiomethyl-, Phenyl-, Benzyl-, 2-Phenylethyl-, p-Hydroxybenzyl-, p-Hydroxyphenyl-, p-Chlorphenyl-, m-Hydroxyphenyl-, Histidinyl-, Imidazolyl-, Triazolyl-, Tetrazolyl-, 2-Thiazolylthio-, Alkinyl-, Vinylrest. Freie Hydroxy-, Thiol- oder Aminogruppen an den Resten R₅ können auch nach dem Fachmann bekannten Methoden (z.B. in P. J. Kocienski, Protecting Groups, Thieme Verlag, 1994, Stuttgart, New York, Seiten 185 - 243) mit einer Schutzgruppe versehen sein. Besonders bevorzugt handelt es sich bei R⁵ um einen Phenylthiomethyl- oder einen Benzylrest.

P¹ und P² sind gleich oder verschieden und bedeuten vorzugsweise Wasserstoffrest oder Amino-Schutzgruppe.

Unter einer Amino-Schutzgruppe ist jede Schutzgruppe zu verstehen, die zum Schutz einer Aminofunktion verwendet werden kann. Solche Schutzgruppen sind bekannt (z.B. in P. J. Kocienski, Protecting Groups, Thieme Verlag, 1994, Stuttgart, New York, Seiten 185 - 243). Beispiele für solche Schutzgruppen sind: Methoxycarbonyl-, Ethoxycarbonyl-, t-Butoxycarbonyl- (BOC), Benzyloxycarbonyl- (Z), Fluorenyloxycarbonyl- (FMOC), Acetyl, Benzyl, Dibenzyl, Phtalimido, Tosyl, Benzoyl oder Silylgruppen wie Trimethylsilyl, Stabase (2,2,5,5-Tetramethyl-1-aza-2,5-disilacyclopentan-Derivate), Benzostabase (2,2,5,5-Tetramethyll-aza-2,5-disila-benzocyclopentan-Derivate).
Bevorzugt handelt es sich um eine t-Butoxycarbonyl- (BOC) oder eine Benzyloxycarbonyl-(Z)-Gruppe.

Vorzugsweise handelt es sich bei dem Carbonsäurederivat (6) um einen optisch aktiven, enantiomerenreinen L- oder D-Aminosäureester. Vorzugsweise ist der optisch aktive, enantiomerenreine L- oder D-Aminosäureester eine Verbindung aus der Gruppe tert-Butoxycarbonyl- oder benzyloxycarbonylgeschützter L-Phenylalaninester oder L-S-Phenylcysteinester.

Die Metall-Enolate der Silane (3) werden durch Umsetzung der Silane mit 2 oder mehr Moläquivalenten von speziellen Basen in Ethern (z. B. Tetrahydrofuran, Methyl-tert.-butyl-ether) erzeugt.
Als spezielle Basen zur Erzeugung des Enolates, bei dem sowohl nur eine als auch beide, gegebenenfalls mit R² substituierte, α-Halogencarbonsäure-Einheiten deprotoniert sein können, sind alle starken Basen wie Alkalimetallbasen oder Erdalkalimetallbasen geeignet. Geeignet sind Organolithiumverbindungen (z.B. n-Butyllithium, sec-Butyllithium, tert.-Butyllithium, Methyllithium, Lithiumdiisopropylamid, Lithiumhexamethyldisilazid, Lithiumcyclohexylamid, Lithiumcyclopentylamid); Kalium-tert.butoxid, Natriumverbindungen wie Natriumhydrid und Natriummethoxid oder -ethoxid sowie analoge Kaliumverbindungen; Magnesiumamide (z.B. Chlormagnesium-diisopropylamid, Brommagnesiumdiisopropylamid, Chlormagnesium-hexamethyldisilazid, Brommagnesium-hexamethyldisilazid, Magnesium-bis(diisopropylamid), Magnesium-bis (hexamethyldisilazid), Chlormagnesium-cyclohexylamid, Brommagnesium-cyclohexylamid) und Organomagnesiumverbindungen (z.B. tert.-Butylmagnesiumchlorid, n-Butylmagnesiumchlorid, Methylmagnesiumchlorid, t-Amylmagnesiumchlorid und entsprechende -bromide; Di-tert.-Butylmagnesium, Diisopropylmagnesium). Werden Organometallverbindungen verwendet, so kann ein Amin wie Diisopropylamin, Hexamethyldisilazan, Triethylamin, Diisopropylethylamin auch vor oder während der Reaktion zugegeben werden.
Das Molverhältnis zwischen Organometallverbindung und Amin beträgt zwischen 0,1 und 10.
Bevorzugte Basen sind Magnesiumverbindungen.
Besonders bevorzugte Basen sind Magnesiumamide oder eine Kombination einer Alkylmagnesiumverbindung mit einem Amin.

Sie werden vorzugsweise dadurch hergestellt, dass ein Magnesiumamid der allgemeinen Formel (8) 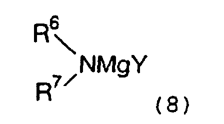 wobei
R⁶ und R⁷ unabhängig voneinander Alkyl-, Aralkyl-, Aryl-, oder Silylrest bedeutet oder R⁶ und R⁷ zusammengenommen einen Cycloalkylring mit 4 bis 6 C Atomen bilden und
Y -NR⁶R⁷ oder Halogenrest bedeutet
oder eine Organomagnesiumverbindung der Formel (9)
R⁸MgZ (9)
wobei
R⁸ Alkyl-, Aryl- oder Aralkylrest und
z Halogenrest oder R³ bedeutet,
oder Gemische der Verbindungen (9), wie sie insbesondere in Grignard-Lösungen (sog. Schlenck-Gleichgewicht) vorliegen,
mit einem Silylester der allgemeinen Formel (3) zur Reaktion bringt.

Im Rahmen dieser Erfindung wird mit Metallenolat allgemein die aktive Spezies bezeichnet, die sich bei der Reaktion zwischen dem Silylester (3) und einer starken Base (Metallkation, z.B. Li oder Mg) in einem geeigneten Lösungsmittel bildet.

Die Bezeichnung "Magnesiumenolat" bezeichnet die aktive Spezies, die sich bei der Reaktion zwischen dem Silylester (3) und einer magnesiumhaltigen Base wie Chlormagnesiumdiisopropylamid in einem geeigneten Lösungsmittel bildet. Dabei kann sowohl nur eine als auch beide, gegebenenfalls substituierten α-Halogencarbonsäureeinheiten deprotoniert werden.
Vorzugsweise bedeuten R⁶ und R⁷ unabhängig voneinander linearer oder verzweigter Alkylrest mit 1 bis 10 Kohlenstoffatomen, Arylrest mit 6 bis 10 Kohlenstoffatomen, Aralkyl mit 7 bis 15 Kohlenstoffatomen, Silylrest mit 3 bis 10 Kohlenstoffatomen. Beispiel für Reste R⁶ und R⁷ sind der Methyl-, Ethyl-, Propyl-, Isopropyl-, tert.-Butyl-, Phenyl-, Benzyl-, Trimethylsilylrest oder R⁶ und R⁷ zusammengenommen -(CH₂)₄- oder -(CH₂)₅-.

Besonders bevorzugt ist R⁶ = R⁷ = Isopropyl- oder Trimethylsilylrest.

R⁸ ist vorzugsweise ein linearer oder verzweigter Alkylrest mit 1 bis 10 Kohlenstoffatomen, ein Arylrest mit 6 bis 10 Kohlenstoffatomen oder ein Aralkylrest mit 7 bis 15 Kohlenstoffatomen. Beispiele für R⁸ sind Methyl-, Ethyl-, Propyl-, Isopropyl-, t-Butyl-, iso-Butyl-, n-Butyl-, Phenyl- oder Benzylrest. Besonders bevorzugt ist R⁸ ein sekundärer oder tertiärer Rest wie z.B. ein iso-Propyl-, sec-Butyl-, t-Butylrest.

Das erfindungsgemäße Verfahren wird vorzugsweise derart durchgeführt, dass man einen Silylester (3) und eine Base gemäß Formel (8) oder (9) und ein Carbonsäurederivat gemäß Formel (2) oder vorzugsweise ein aktiviertes (Amino)-Säurederivat gemäß Formel (6) vorzugsweise bei 0°C bis Raumtemperatur in einem der oben genannten Lösungsmittel zusammengibt und die Lösung entweder bei Raumtemperatur bis zum vollständigen Umsatz rührt, oder für 10-180 min auf 40-110°C erhitzt und nach vollständiger Umsetzung die Lösung, die das Additionsprodukt der Reaktion enthält, mit verdünnter Säure versetzt wird, wodurch das Produkt (1) bzw. (5) in-situ freigesetzt wird und diese Mischung mit einem mit Wasser nicht mischbaren organischen Lösungsmittel extrahiert wird und eine halogenketonhaltige organische Phase abgetrennt und die halogenketonhaltige organische Phase vorzugsweise mit einer gesättigten NaHCO₃- oder NaCl-Lösung gewaschen und vorzugsweise über Na₂SO₄ oder MgSO₄ getrocknet wird, das Lösungsmittel abgezogen wird, ein gebildetes Disiloxan entfernt wird und das α-Halogenketon als kristalliner Rückstand vorliegt.

Das Molverhältnis der eingesetzten Base zum verwendeten Silylester beträgt vorzugsweise zwischen 1 und 8, besonders bevorzugt zwischen 2 und 6.
Das Molverhältnis des Silylesters zum Carbonsäurederivat beträgt vorzugsweise zwischen 0,5 und 2, besonders bevorzugt zwischen 0,5 und 1,5.

Die Reaktionszeit beträgt bei Raumtemperatur zwischen 1 h und 16 h, bei Erwärmen zwischen 10 min und 3 h. Das Erwärmen auf vorzugsweise 40 bis 110°C, besonders bevorzugt 40 bis 70°C ist aufgrund der Reaktionsbeschleunigung die bevorzugte Ausführungsvariante. Unter Raumtemperatur ist vorzugsweise eine Temperatur von 20 bis 25°C zu verstehen.
Die vollständige Umsetzung ist bei Verwendung von Chlormagnesium-diisopropylat als Base einfach am Klären der Suspension zu einer Lösung zu erkennen.
Die sich an die eigentliche Reaktion unmittelbar anschließende Aufarbeitungsprozedur kann allgemein wie folgt beschrieben werden: Der Reaktionsansatz wird in verdünnte Säure getropft. Prinzipiell ist nur ein pH-Bereich auf sauer bis neutral zu erreichen. Beispiele für die verwendbaren Säuren sind Salzsäure (2 bis 30%ig) und Schwefelsäure (2 bis 20%ig) und ähnliches wie gesättigte Ammoniumchloridlösung oder verdünnte Essigsäure. Beim Ansäuern wird das Produkt (1) bzw. (5) in-situ freigesetzt, da bei Wasserkontakt unmittelbar hintereinander sowohl die Hydrolyse des Silylesters und die nachfolgende Decarboxylierung zum Halogenketon erfolgen. Nach Erreichen eines sauren bis neutralen pH-Wertes von < 7, bevorzugt 1 bis 4, und beendeter Gasentwicklung wird die erhaltene Mischung mit einem mit Wasser nicht mischbaren organischen Lösungsmittel extrahiert. Bei dem mit Wasser nicht mischbaren organischen Lösungsmittel handelt es sich vorzugsweise um Essigsäureester wie Ethylacetat, Propylacetat oder Butylacetat, Ether wie t-Butyl-methylether. Tetrahydrofuran, Dioxan, Diethylether, Diisopropylether, Dibutylether oder 1,2-Dimethoxyethan oder aromatische Kohlenwasserstoffe wie Toluol, Xylol oder Benzol. Prinzipiell ebenso möglich sind halogenierte Kohlenwasserstoffe wie Dichlormethan oder Chloroform. Aufgrund der höheren Dichte ergeben sich hierbei jedoch erschwerte Probleme bei der Phasentrennung und es muss mit mehr Extraktionsmittel gearbeitet werden. Die halogenketonhaltige organische Phase wird dann mit gesättigter NaHCO₃und/oder NaCl-Lösung gewaschen und über Na₂SO₄ oder MgSO₄ getrocknet. Nach Abfiltrieren des Trockenmittels und Abziehen des Lösungsmittels wird das gebildete Oligo- bzw. Polysiloxan durch Ausrühren mit Petrolbenzin abgetrennt. Der feste Rückstand des α-Halogenketones kann entweder direkt in die Reduktion zum α-Halogenalkohol eingeführt werden oder nach Umkristallisation. Besonders geeignete Lösungsmittel zur Umkristallisation sind Alkohole wie Ethanol, 2-Propanol, 2-Butanol, Hexanol, 2-Ethylhexanol. Diese können auch als Gemische mit Petrolbenzin eingesetzt werden. Bei der Umkristallisation wird auch das als Kuppelprodukt anfallende Siloxan -[Si(CH3)₂O]- entfernt, sodass das Ausrühren mit Petrolbenzin unterbleiben kann.

Die Reduktion zum α-Halogenalkohol kann durch Reduktion des α-Halogenketons mit NaBH₄ unter den in S. W. Kaldor et al., J. Med. Chem. 1997, 40, 3979-3985, beschriebenen Bedingungen geschehen, oder besonders vorteilhaft aufgrund der ausgeprägten Diastereoselektivität durch Meerwein-Ponndorf-Verley-(MPV)-Reduktion wie beispielsweise in EP 963972, S. 13, Z.42 bis S. 14, Z. 11. beschrieben erfolgen.

Als exemplarisches Beispiel für die Kombinationsmöglichkeiten von Substraten und Reagenzien der vorliegenden Erfindung dient folgendes Schema:

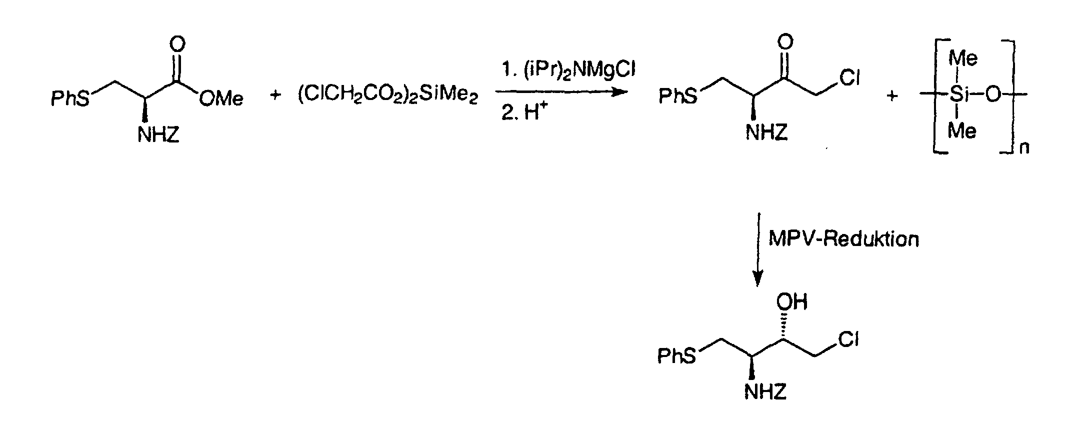

Die vorliegende Erfindung umfasst somit auch die Herstellung eines α-Halogenalkohols der allgemeinen Formel (4) 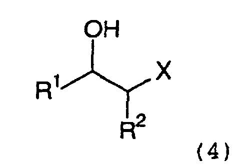 wobei R¹, R² und X wie oben definiert sind
durch Herstellung eines α-Halogenketons (1) nach dem erfindungsgemäßen Verfahren und Reduktion der Carbonylgruppe zum α-Halogenalkohol (4).

Die Erfindung umfasst insbesondere auch die Herstellung eines α-Halogenalkohols der allgemeinen Formel (7) 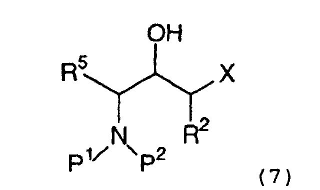 wobei R², R⁵, P¹, P², und X die bereits genannte Bedeutung, haben, durch Herstellung eines α-Halogenketons (5) nach dem erfindungsgemäßen Verfahren und Reduktion der Carbonylgruppe zum α-Halogenalkohol (7).

Das erfindungsgemäße Verfahren ermöglicht einen vorteilhaften kostengünstigen Zugang zu α-Halogenketonen ausgehend von (N-geschützten Amino)-Säurederivaten. Ausgehend von L-Phenylalanin und L-Phenylcystein werden daraus durch Reduktion der Ketogruppe die entsprechenden Halogenalkohole erhalten, die weiter zu HIV Protease Hemmern umgesetzt werden können (S. W. Kaldor et al., J. Med. Chem. 1997, 40, 3979-3985; K.-J. Schleifer, Pharmazie in unserer Zeit 2000, 29, 341 - 349).

Zudem kann die Reinigung durch einfache Umkristallisation erfolgen, eine technisch nicht durchführbare Säulenchromatographie ist nicht notwendig.

Die folgenden Beispiele dienen der weiteren Erläuterung der Erfindung.

### Beispiel 1:

Chloressigsäure-Natriumsalz (233 g, 2.00 mol) wird in abs. Diethylether (400 ml) suspendiert. Unter Rühren wird Dichlordimethylsilan (122 ml, 1.00 mol) zugetropft. Die Reaktionsmischung wird zur Vervollständigung der Reaktion für 60 min zum Rückfluß erhitzt. Nach Abkühlen wird das gebildete NaCl unter Schutzgasatmosphäre abfiltriert. Das quantitativ anfallende Rohprodukt wird durch Abziehen des Ethers isoliert und ist für die nachfolgend beschriebene Umsetzung hinreichend rein. Zur weiteren Reinigung für analytische Zwecke kann das Rohprodukt fraktionierend destilliert werden. Farblose Flüssigkeit (164 g, 67 %), Sdp. 90°C / 1 mbar. ¹H-NMR (CDCl₃): 0.50 (s, 6H), 4.08 (s, 4H). ²⁹Si-NMR: 8.88 ppm.

### Beispiel 2:

Diisopropylamin (21 ml, 150 mmol) wird bei RT unter Rühren zu Methylmagnesiumchlorid (58 ml, 20% in Tetrahydrofuran (THF), 150 mmol) getropft, die Suspension mit abs. THF (30 ml) verdünnt und für 60 min zum Rückfluß erhitzt (Kolben 1). In einem zweiten Kolben werden N-Z-L-S-Phenylcysteinmethylester (8,6 g, 25 mmol) und Bis-(chloracetoxy)-dimethylsilan (6,1 g, 25 mmol) unter Rühren bei RT in abs. THF (40 ml) gelöst (Kolben 2). Die Suspension aus dem ersten Kolben wird nun binnen 5 Minuten zur Lösung in Kolben 2 getropft. Dabei tritt eine stark exotherme Reaktion ein und es entsteht eine gelbe Suspension. Die Mischung wird noch für 45 min am Rückfluß gehalten. Während dieser Zeit entsteht aus der Suspension eine klare Lösung. Das Reaktionsgemisch wird mit THF (100 ml) verdünnt, in verdünnte Schwefelsäure (10%) getropft, mit voll entsalztem oder destilliertem Wasser und gesättigter NaCl-Lösung gewaschen. Die organische Phase wird am Rotationsverdampfer eingeengt. Der Rückstand kristallisiert rasch durch und wird mit Petrolether ausgerührt. Die hellbeigen Kristalle werden abgesaugt und im Trokkenschrank getrocknet. Ausbeute 6,8 g (74%). Umkristallisation aus 2-Propanol. Schmp. 90 - 91°C. ¹H-NMR (CDCl₃): 3.22 - 3.45 (m, 2H), 4.10 - 4.25 (m, 2H), 4.63 - 4.75 (m, 2H), 5.10 (s, 2H), 5.50 - 5.65 (m, 1H), 7.15 - 7.45 (m, 10H).
Alternative Aufarbeitung: Durch Aufkonzentrieren der Lösung und direkten Zusatz von iso-Propanol kann das Produkt auch direkt aus der Reaktionsmischung kristallin erhalten werden wobei die nicht optimierte Ausbeute 5,1 g (56 %) beträgt.

### Beispiel 3:

Methylmagnesiumchlorid-Lösung (20% in THF, 300 mmol, 116 ml) wurde.vorgelegt, zum Sieden erhitzt und tropfenweise mit Diisopropylamin (300 mmol, 42.0 ml) versetzt. Nach Zugabe wurde noch 1 h refluxiert, dann abgekühlt.
N-Z-L-Phenylalaninmethylester (37.5 mmol, 11.7 g) und
Bis(chloracetoxy)-dimethylsilan (50 mmol, 15,7 g) wurden in THF (40 ml) gelöst und auf 0°C gekühlt. Die hergestellte Suspension des Chlormagnesiumdiisopropylamids wurde nun derart zugetropft, dass die Temperatur nicht über 30°C stieg. Die erhaltene gelbe Suspension wurde für 30 min zum Rückfluß erhitzt. Dann wurde abgekühlt und die braune Lösung zu 20% Salzsäure (85 ml) getropft. Die Phasen werden getrennt und die organische aufkonzentriert. Der Rückstand wird aus Isopropanol umkristallisiert. Es wurden 9,56 g (78%) leicht gelbliche Kristalle erhalten (Reinheit HPLC 99,7%), Schmp. 91-94°C, ¹H-NMR (CDCl₃): δ 2.95-3.15 (m, 2H, PhC*H*₂CH(NHZ)CO), 3.95 (d, 1H, C*H*HCl), 4.15 (d, 1 H, CH*H*Cl), 4.70-4.80 (m, 1 H, CH₂C*H*(NHZ)CO), 5.05 (s, 2 H, PhC*H*₂O), 5.20-5.35 (bs, 1 H, N*H*Z), 7.10-7.20 (m, 2 H, aromat.), 7.25-7.40 (m, 8 H, aromat.).

### Beispiel 4:

Methylmagnesiumchlorid-Lösung (20% in THF, 100 mmol, 39 ml) wurde vorgelegt, zum Sieden erhitzt und tropfenweise mit Diisopropylamin (100 mmol, 14.0 ml) versetzt. Nach Zugabe wurde noch 1 h refluxiert, dann abgekühlt.

Benzoesäuremethylester (17 mmol, 2,30 g) und Bis(chloracetoxy)-dimethylsilan (17 mmol, 5.33 g) wurden in THF (20 ml) gelöst und auf +5°C gekühlt. Die hergestellte Suspension des Chlormagnesiumdiisopropylamids wurde nun derart zugetropft, dass die Temperatur nicht über 30°C stieg. Die erhaltene Suspension wurde für 60 min zum Rückfluß erhitzt. Dann wurde abgekühlt und die braune Lösung zu 20% Salzsäure (40 ml) getropft. Die Phasen werden getrennt und die organische aufkonzentriert. Der Rückstand wurde per HPLC analysiert. Es wurden durch Retentionszeitenvergleich mit käuflichem Phenacylchlorid 89,4% Produkt und 6,3% Edukt bestimmt, Rest unbekannt.

### Beispiel 5:

Methylmagnesiumchlorid-Lösung (20% in THF, 300 mmol, 116 ml) wurde vorgelegt, zum Sieden erhitzt und tropfenweise mit Diisopropylamin (300 mmol, 42.0 ml) versetzt. Nach Zugabe wurde noch 1 h refluxiert, dann abgekühlt.
Chloressigsäurenatriumsalz (100 mmol, 11.6 g) und Dichlordimethylsilan (50 mmol, 6,1 ml) wurden in absolutem THF (50 ml) für 2h auf 40°C erhitzt. Pivalinsäuremethylester (50 mmol, 6,70 ml) wurde zugesetzt und die Reaktionsmischung auf +5°C gekühlt. Die hergestellte Suspension des Chlormagnesiumdiisopropylamids wurde nun derart zugetropft, dass die Temperatur nicht über 30°C stieg. Die erhaltene Suspension wurde für 60 min zum Rückfluß erhitzt. Dann wurde abgekühlt und die braune Lösung zu 12% Salzsäure (100 ml) getropft. Die Phasen werden getrennt. Die organische Phase wurde per GC analysiert. Gehalt an 1-Chlor-3,3-dimethyl-2-butanon 91,2%, Edukt 4,3%. Das Lösungsmittel wurde über eine Kolonne abdestilliert und der Rückstand fraktionierend destilliert. Es wurden 5,10 g (76%) Hauptfraktion erhalten (GC > 95%), Sdp. 75 - 80°C/18 mbar, ¹H-NMR (CDCl₃): δ 1.20 (s, 9 H, tBu), 4.40 (s, 2 H, COC*H*₂Cl).

## Patentansprüche

1. Verfahren zur Herstellung eines α-Halogenketons der allgemeinen Formel (1) 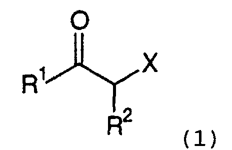 wobei R¹ ein Alkyl-, Aralkyl- oder Arylrest ist, bei dem gegebenenfalls CH₂-Einheiten durch Heteroatome wie NH, NCH₃, S, O substituiert sein können und gegebenenfalls CH-Einheiten durch N substituiert sein können, und die Reste R¹ ferner mit funktionellen Gruppen, wie z. B. einem Halogenrest, einem Aminorest, einem Alkoxyrest oder einem Thioalkylrest substituiert sein können und
R² ein Wasserstoff-, Alkyl-, Aralkyl- oder Arylrest ist und
X ein Halogenrest ist
in welchem ein Carbonsäurederivat der allgemeinen Formel (2) 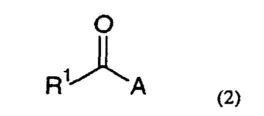 wobei R¹ die bereits genannte Bedeutung hat und
A eine Abgangsgruppe ist,
mit einem Mono- oder Dienolat eines Silylesters der allgemeinen Formel (3) 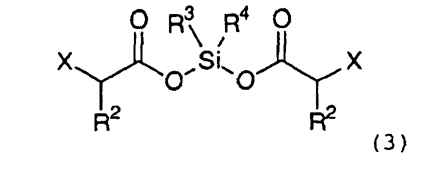 wobei X und R² die bereits genannte Bedeutung haben und
R³, R⁴ gleich oder verschieden sind und Alkyl-, Aryl-, Alkenyl- oder Aralkylrest bedeuten; zur Reaktion gebracht wird und das Reaktionsprodukt unmittelbar anschließend durch Säurezusatz hydrolysiert und zu (1) decarboxyliert wird.

2. Verfahren gemäß Anspruch 1 zur Herstellung eines α-Halogenketons der allgemeinen Formel (5) 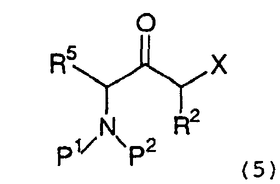 wobei R² wie oben definiert ist und R⁵ Wasserstoff-, Alkyl-, Aralkyl- oder Arylrest bedeutet, bei dem eine oder mehrere CH₂-Gruppen durch O, S, NH, NCH₃ substituiert sein können, und
X ein Halogenrest ist und
P¹, und P² unabhängig voneinander Wasserstoffrest oder eine Amino-Schutzgruppe sind, bzw. zusammengenommen eine cyclische Aminoschutzgruppe sind,
bei dem ein Aminosäurederivat der allgemeinen Formel (6) 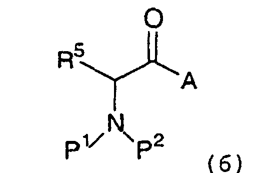 wobei R⁵, P¹, P² die bereits genannte Bedeutung haben und A eine Abgangsgruppe ist,
mit einem Metall Mono- oder Dienolat eines Silylesters der allgemeinen Formel (3) zur Reaktion gebracht wird und das Reaktionsprodukt anschließend unmittelbar durch einen Säurezusatz hydrolysiert und zu (5) decarboxyliert wird.

3. Verfahren gemäß Anspruch 1 oder 2, **dadurch gekennzeichnet, dass** es bei einer Temperatur im Bereich von -10°C bis 110°C, bevorzugte bei 0°C bis 70°C durchgeführt wird.

4. Verfahren gemäß einem der Ansprüche 1 bis 3, **dadurch gekennzeichnet, dass** es sich bei der zur Erzeugung des Enolates des Silylesters benutzten Base um ein Magnesiumamid oder eine Kombination einer Alkylmagnesiumverbindung mit einem Amin handelt.

5. Verfahren gemäß einem der Ansprüche 1 bis 4, **dadurch gekennzeichnet, dass** das Molverhältnis der eingesetzten Base zum verwendeten Bissilylester zwischen 2 und 8, besonders bevorzugt zwischen 2 und 6 beträgt.

6. Verfahren gemäß einem der Ansprüche 1 bis 5, **dadurch gekennzeichnet, dass** das Molverhältnis des Bissilylesters zum Carbonsäurederivat vorzugsweise zwischen 0,5 und 2, besonders bevorzugt zwischen 0,5 und 1,5 beträgt.

7. Verfahren gemäß einem der Ansprüche 2 bis 6, **dadurch gekennzeichnet, dass** das Carbonsäurederivat (6) ein optisch aktiver, enantiomerenreiner L- oder D-Aminosäureester ist.

8. Verfahren gemäß Anspruch 7, **dadurch gekennzeichnet, dass** der optisch aktive, enantiomerenreine L- oder D-Aminosäureester eine Verbindung aus der Gruppe tert.-Butoxycarbonyl- oder benzyloxycarbonylgeschützter L-Phenylalaninester oder L-S-Phenylcysteinester ist.

9. Verfahren zur Herstellung eines α-Halogenalkohols der allgemeinen Formel (4) 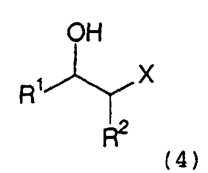 wobei R¹, R² und X wie genannt definiert sind
durch Herstellung eines α-Halogenketons (1) nach einem Verfahren gemäß einem der Ansprüche 1 bis 8 und anschließender Reduktion der Carbonylfunktion zum α-Halogenalkohol (4).

10. Verfahren zur Herstellung eines α-Halogenalkohols der allgemeinen Formel (7) 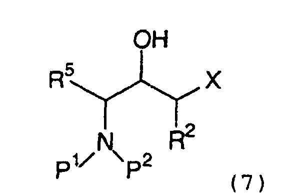 wobei R², R⁵, P¹, p², und X die bereits genannte Bedeutung haben,
durch Herstellung eines α-Halogenketons (5) nach einem Verfahren gemäß einem der Ansprüche 2 bis 8 und anschließender Reduktion der Carbonylgruppe zum sekundären Alkohol (7).

## Claims

1. Process for preparing an α-haloketone of the general formula (I) 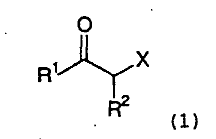 where R¹ is an alkyl, aralkyl or aryl radical in which CH₂ units may be replaced by heteroatoms such as NH, NCH₃, S or O and CH units may be substituted by N, and the R¹ radicals may further be replaced by functional groups, for example a halogen radical, an amino radical, an alkoxy radical or a thioalkyl radical and
R² is a hydrogen, alkyl, aralkyl or aryl radical and
X is a halogen radical,
by reacting a carboxylic acid derivative of the general formula (2) 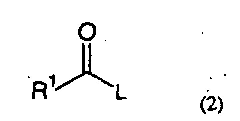 where R¹ is as defined above and
L is a leaving group,
with a mono- or dienolate of a silyl ester of the general formula (3) 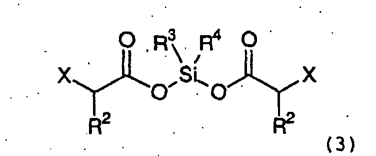 where X and R² are each as defined above and R³ and
R⁴ are identical or different and are each an alkyl, aryl, alkenyl or aralkyl radical;
and hydrolysing the reaction product immediately afterwards by adding acid and decarboxylating it to (1).

2. Process according to Claim 1 for preparing an α-haloketone of the general formula (5) 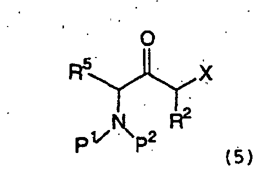 where R² is as defined above and R⁵ is a hydrogen, alkyl, aralkyl or aryl radical in which one or more CH₂ groups may be replaced by O, S, NH or NCH₃,
and
X is a halogen radical and
P¹ and P² are each independently a hydrogen radical or an amino protecting group, or are together a cyclic amino protecting group,
by reacting an amino acid derivative of the general formula (6) 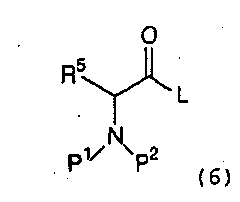 where R⁵, P¹ and P² are each as defined above and
L is a leaving group,
with a metal mono- or dienolate of a silyl ester of the general formula (3) and hydrolysing the reaction product immediately afterwards by adding acid and decarboxylating it to (5).

3. Process according to Claim 1 or 2, **characterized in that** it is carried out at a temperature in the range from -10°C to 110°C, preferably from 0°C to 70°C.

4. Process according to any of Claims 1 to 3, **characterized in that** the base used to generate the enolate of the silyl ester is a magnesium amide or a combination of an alkyl magnesium compound with an amine.

5. Process according to any of Claims 1 to 4, **characterized in that** the molar ratio of the base used to the bissilyl ester used is from 2 to 8, more preferably from 2 to 6.

6. Process according to any of Claims 1 to 5, **characterized in that** the molar ratio of the bissilyl ester to the carboxylic acid derivative is preferably from 0.5 to 2, more preferably from 0.5 to 1.5.

7. Process according to any of Claims 2 to 6, **characterized in that** the carboxylic acid derivative (6) is an optically active; enantiomerically pure L- or D-amino ester.

8. Process according to Claim 7, **characterized in that** the optically active, enantiomerically pure L- or D-amino ester is a compound from the group of tert-butoxycarbonyl- or benzyloxycarbonyl-protected L-phenylalanine esters or L-S-phenylcysteine esters.

9. Process for preparing an α-haloalcohol of the general formula (4) 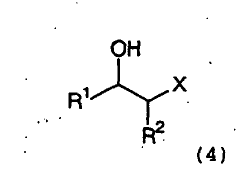 where R¹, R² and X are each as defined above by preparing an α-haloketone (1) by a process according to any of Claims 1 to 8 and subsequently reducing the carbonyl function to the α-haloalcohol (4).

10. Process for preparing an α-haloalcohol of the general formula (7) 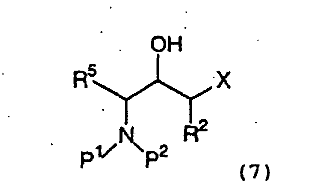 where R², R⁵, P¹, P² and X are each as defined above,
by preparing an α-haloketone (5) by a process according to any of Claims 2 to 8 and subsequently reducing the carbonyl group to the secondary alcohol (7).

## Revendications

1. Procédé pour la préparation d'une cétone α-halogénée de formule générale (1) 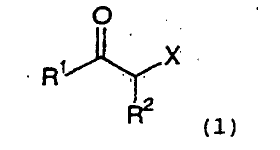 R¹ étant un radical alkyle, aralkyle ou aryle dans lequel des groupes CH₂ peuvent éventuellement être remplacés par des hétéroatomes tels que NH, NCH₃, S, O et des groupes CH peuvent éventuellement être remplacés par N, et les radicaux R¹ peuvent en outre être substitués par des groupes fonctionnels, comme par exemple un radical halogéno, un radical amino, un radical alcoxy ou un radical thioalkyle et
R² étant un atome d'hydrogène, un radical alkyle, aralkyle ou aryle et
X étant un radical halogéno,
dans lequel on fait réagir un dérivé d'acide carboxylique de formule générale (2) 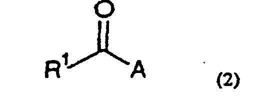 dans laquelle R¹ a la signification déjà donnée et A est un groupe partant, avec un mono- ou diénolate d'un ester silylique de formule générale (3) 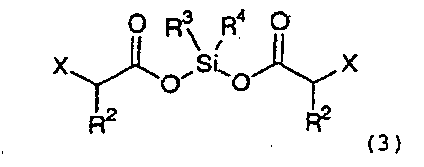 dans laquelle X et R² ont les significations déjà données et
R³, R⁴ sont identiques ou différents et représentent un radical alkyle, aryle, alcényle ou aralkyle ;
et le produit de réaction est ensuite immédiatement hydrolysé par addition d'un acide et décarboxylé pour donner (1).

2. Procédé pour la préparation d'une cétone α-halogénée de formule générale (5) 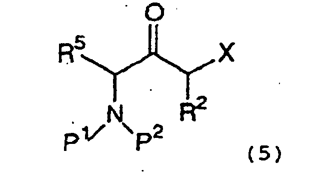 R² étant tel que défini plus haut et R⁵ représentant un atome d'hydrogène, un radical alkyle, aralkyle ou aryle, dans lequel un ou plusieurs groupes CH₂ peuvent être remplacés par O, S, NH, NCH₃ et
X est un radical halogéno et
P¹ et P² représentent, indépendamment l'un de l'autre, un atome d'hydrogène ou un groupe protecteur de fonction amino, ou forment ensemble un groupe cyclique protecteur de fonction amino,
dans lequel on fait réagir un dérivé d'aminoacide de formule générale (6) 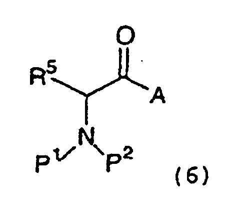 dans laquelle R⁵, P¹, P² ont les significations déjà données et
A est un groupe partant,
avec un mono- ou diénolate métallique d'un ester silylique de formule générale (3) et le produit de réaction est ensuite immédiatement hydrolysé par addition d'un acide et décarboxylé pour donner (5).

3. Procédé selon la revendication 1 ou 2, **caractérisé en ce qu'**il est effectué à une température dans la plage de -10°C à 110°C, de préférence à 0-70°C.

4. Procédé selon l'une quelconque des revendications 1 à 3, **caractérisé en ce que** la base utilisée pour la production de l'énolate de l'ester silylique consiste en un amidure de magnésium ou une association d'un composé de type alkylmagnésium avec une amine.

5. Procédé selon l'une quelconque des revendications 1 à 4, **caractérisé en ce que** le rapport molaire de la base utilisée à l'ester bis-silylique utilisé est compris entre 2 et 8, de façon particulièrement préférée entre 2 et 6.

6. Procédé selon l'une quelconque des revendications 1 à 5, **caractérisé en ce que** le rapport molaire de l'ester bis-silylique au dérivé d'acide carboxylique est compris de préférence entre 0,5 et 2, de façon particulièrement préférée entre 0,5 et 1,5.

7. Procédé selon l'une quelconque des revendications 2 à 6, **caractérisé en ce que** le dérivé d'acide carboxylique (6) est un ester d'aminoacide D ou L optiquement actif, sous forme d'énantiomère pur.

8. Procédé selon la revendication 7, **caractérisé en ce que** l'ester d'aminoacide D ou L optiquement actif, sous forme d'énantiomère pur, est un composé choisi parmi les esters de L-phénylalanine et les esters de L-S-phénylcystéine protégés par le groupe tert-butoxycarbonyle ou benzyloxycarbonyle.

9. Procédé pour la préparation d'un alcool α-halogéné de formule générale (4) 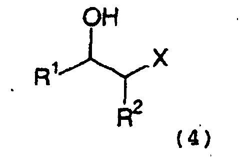 dans laquelle R¹, R² et X sont tels que définis précédemment,
par préparation d'une cétone α-halogénée (1) selon un procédé conforme à l'une quelconque des revendications 1 à 8, et réduction subséquente de la fonction carbonyle en l'alcool α-halogéné (4).

10. Procédé pour la préparation d'un alcool α-halogéné de formule générale (7) 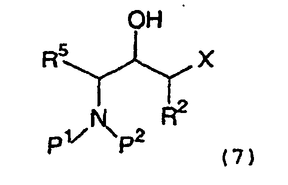 dans laquelle R², R⁵, P¹, P² et X ont les significations déjà données,
par préparation d'une cétone α-halogénée (5) selon un procédé conforme à l'une quelconque des revendications 2 à 8, et réduction subséquente du groupe carbonyle en l'alcool secondaire (7).
